# EUROPÄISCHE PATENTSCHRIFT

(11) **EP 2 348 098 B1**
(45) Veröffentlichungstag und Bekanntmachung des Hinweises auf die Patenterteilung: **23.11.2016**
(21) Anmeldenummer: 10450003.8
(22) Anmeldetag: 20.01.2010
(51) Int. Cl.: C12M 1/107, C12M 1/113

(54) **Erdbeckenfermenter mit einem abgesenkten Beckenboden**
Ground basin fermenter with a submerged basin base
Fermenteur à bassin enterré doté d'un fond de bassin rabaissé

(43) Veröffentlichungstag der Anmeldung: 27.07.2011
(73) Patentinhaber: Sattler AG, 8041 Graz (AT)
(72) Erfinder: Wiedau, Helmut, 48161 Münster (DE)
(74) Vertreter: Ellmeyer, Wolfgang

(56) Entgegenhaltungen:
- EP-A1- 1 992 683
- WO-A1-2009/155629
- WO-A2-2009/073902
- DE-A1-102005 061 039
- DE-U1-202004 005 238
- US-A- 3 933 628
- US-A- 4 439 315

## Beschreibung

Die Erfindung betrifft einen Erdbeckenfermenter mit einem abgesenkten Beckenboden, der mit einer Dichtungsschicht abgedeckt ist und ein Erdbecken ausbildet, und an dessen Beckenkrone zumindest eine gasdichte Abdeckung festgelegt ist, wobei Zu- und Ableitungen und zumindest ein Rührwerk vorgesehen sind.

Die in der Landwirtschaft seit einiger Zeit unternommenen Anstrengungen zur Verwertung der bei der Nutztierhaltung anfallenden Ausscheidungen oder von pflanzlichen Abfällen führten zur Entwicklung von Fermentationsanlagen, mit denen sogenanntes Biogas gewonnen werden kann.

Der Fermentationsvorgang kann durch Erwärmen des zu fermentierenden Mediums beschleunigt werden und durch die über ein Rührwerk erreichte Zwangsbewegung des Mediums wird dessen Durchmischung sichergestellt, um eine Verklumpung des Mediums sowie Schichten- oder Schlackenbildung zu vermeiden, welche die Effizienz der Fermentation vermindern würde.

Bei großen Beckendurchmessern kann selbst bei Einsatz von mehreren, an unterschiedlichen Stellen angebrachten Rührwerken keine für eine Durchmischung ausreichende Medienströmung erzeugt werden. Alle von den Rührwerken od dgl bewirkten Verwirbelungen führen nicht zu einer gerichteten Strömung, sondern es wird lediglich sehr lokal für eine Medienbewegung gesorgt, während der Großteil des Mediums im Erdbeckens davon unberührt und unbewegt bleibt.

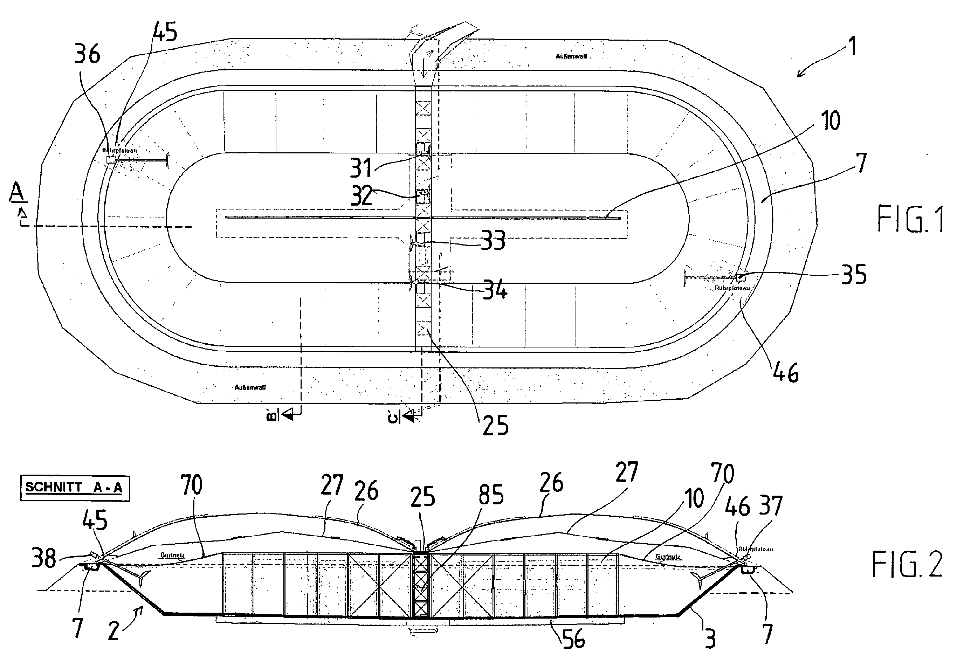

WO2009155629 A offenbart einen Erdbeckenfermenter mit einem abgesenkten Beckenboden, der mit einer Dichtungsschicht abgedeckt ist und ein Erdbecken ausbildet. Zu- und Ableitung sind oberhalb des Beckenbodens und innerhalb der Beckenkrone geführt. Fig. 1 zeigt einen Erdbeckenfermenter mit einem abgesenkten Beckenboden, seitlicher Wandschrägen sowie mindestens ein Rührwerk.

Aufgabe der Erfindung ist es, einen eingangs genannten Erdbeckenfermenter anzugeben, der mit geringem Energieeinsatz eine ausreichende Durchmischung und Homogenisierung des im Erdbecken befindlichen Mediums, insbesondere für großvolumige Erdbecken ermöglicht.

Weitere Aufgabe der Erfindung ist es, eine weitere Möglichkeit des Wärmeeintrags in das zu fermentierende Medium zu schaffen.

Weitere Aufgabe der Erfindung ist es, einen Erdbeckenfermenter anzugeben, der strömungstechnisch verbessert ist und zugleich eine Auflagerstelle im Inneren des Erdbeckens bietet.

Erfindungsgemäß wird dies dadurch erreicht, dass innerhalb des Erdbeckens zumindest eine Strömungsleitwand vorgesehen ist, welche entlang eines Abschnitts eine Unterteilung des Querschnitts des Erdbeckens bewirkt, sodass bei Betätigung des zumindest einen Rührwerks zu beiden Seiten der Strömungsleitwand sich eine Medienströmung ausbildet, wobei sich gemäß Anspruch 1 vom Beckenboden Wandschrägen zu der umlaufenden Bckenkrone erstrecken.

Durch das Ausbilden einer Strömungsleitwand oder von mehreren Strömungsleitwänden verkleinert sich ein Teil des Beckenquerschnitts derart, dass z.B. ein kanalförmiger Bereich entsteht, wodurch die in diesem Bereich einwirkenden Rührgeräte bei gleicher Energiezufuhr eine höhere Strömungsgeschwindigkeit erzeugen können, welche wiederum Voraussetzung dafür ist, dass vorhandene Materialschichten innerhalb des Mediums durchbrochen oder Klumpen aufgelöst werden. Diese Verkleinerung des Querschnitts kann auf beiden Seiten der Strömungsleitwand genutzt werden.

Die erfindungsgemäß ausgebildete Strömungsleitwand kann aus entsprechend geeignetem Material aufgebaut sein, z.B. eine in Stahlbetonbauweise hergestellte Wand oder eine aus Betonfertigteilen zusammengesetzte Wand, die auf dem Beckengrund des erfindungsgemäßen Erdbeckenfermenters errichtet wird. Als Baumaterial für die Strömungsleitwand können aber auch Kunststoff, Holz oder andere Naturstoffe sowie Metalle, z.B. Stahl zur Anwendung gelangen, solange die erforderliche Materialbeständigkeit und Festigkeit gegeben ist. Weiters könnte die Strömungsleitwand z.B.aus einem sehr dichten, gespannten Netz bestehen, sofern keine allzu große Medienbewegung durch das Netz hindurch erfolgen kann.

Eine einfache Ausführungsform der Erfindung kann darin bestehen, dass die Strömungsleitwand vertikal ist. Es können aber auch andere Bauformen, z.B. in Form von schräg verlaufenden Wänden angewendet werden, um einen Teil des Beckenquerschnitts abzuteilen und für die Erzeugung einer Medienströmung bereitzustellen.

Um das Auftreten von ungemischten Schwimmschichten im Erdbecken zu vermeiden, ist es vorteilhaft, wenn gemäß einer weiteren Ausführungsform der Erfindung die Höhe der Strömungsleitwand größer als der maximale Füllstand im Erdbecken ist. Auf diese Weise wird der jeweilige Beckenquerschnitt über die gesamte Höhe unterteilt und gemischt.

Die Orientierung und der Verlauf der Strömungsleitwand kann den Gegebenheiten angepasst werden, es können z.B. mehrere kurze oder aber auch gekrümmte Strömungsleitwände innerhalb des Erdbeckens errichtet sein. Es hat sich gezeigt, dass eine Unterteilung über einen relativ großen Abschnitt des Erdbeckens sich auf die entstehende Strömung zu beiden Seiten der Strömungsleitwand positiv auswirkt, weshalb gemäß einem weiteren Ausführungsbeispiel der Erfindung die zumindest eine Strömungsleitwand sich in Längsrichtung des Erdbeckens erstreckt.

Für die Durchmischung als besonders günstig hat es sich herausgestellt, am Ende der erfindungsgemäß vorgesehenen Strömungsleitwand freie Querschnitte als Umkehrbereiche vorzusehen, in denen die auf der einen Wandseite sich einstellende Strömung in die Gegenrichtung umgelenkt und entlang der anderen Wandseite weitergeleitet wird.

In Weiterbildung der Erfindung kann daher vorgesehen sein, dass die zumindest eine Strömungsleitwand durch eine Strömungsleitwand ausgebildet ist, die sich entlang der Hauptachse des Erdbeckens erstreckt und den Querschnitt des Erdbeckens in zwei gleiche Teile unterteilt, wobei zwischen den Längsenden der Strömungsleitwand und dem in Längsrichtung jeweils gegenüberliegenden Beckenwandbereich ein Strömungsquerschnitt freigestellt ist. Die Unterteilung in zwei gleich große Teilquerschnitte bewirkt die Entstehung einer konstanten Strömung auf beiden Seiten der Strömungsleitwand.

Weiters kann vorgesehen sein, dass das zumindest eine Rührwerk durch jeweils zwei Tauch-Rührwerke zu beiden Seiten der Strömungsleitwand ausgebildet ist, welche mit ihren Rotationsachsen parallel zur Strömungsleitwand bzw. zur Hauptachse ausgerichtet sind. Durch die Parallelstellung der Rührwerk-Rotationsachsen in Bezug auf die Strömungsleitwand wird eine parallel zur Strömungsleitwand orientierte Strömung erzeugt, die auf der gegenüberliegenden Wandseite in der Gegenrichtung verläuft.

Um die Durchmischung im Erdbecken über die gesamte Höhe zu fördern, kann gemäß einer weiteren Ausbildung der Erfindung vorgesehen sein, dass die Tauch-Rührwerke innerhalb des Erdbeckens auf Stützen höhenverstellbar angeordnet sind, die mit ihren oberen Enden auf einer Bedienbrücke aufgelagert sind, welche Bedienbrücke sich entlang der Nebenachse des Erdbeckens über das Erdbecken spannt und Teil der gasdichten Abdeckung bildet. Durch die für die Höhenverstellbarkeit der Rührwerke auf der Bedienbrücke aufgelagerten Stützen kann auch während des Betriebs des Erdbeckenfermenters über einen Serviceschacht eine Wartung oder Reparatur der Rührwerke vorgenommen werden.

Eine für die Durchmischung des Mediums vorteilhafte Bauform kann in einem langlochartigen Grundriss des Erdbeckens bestehen, wobei die Beckenkrone sich an beiden Längsseiten des Erdbeckens parallel erstreckt und an den beiden Schmalseiten durch Halbkreise abgeschlossen ist, und dass in den Halbkreisbereichen weitere Rührwerke jeweils zwischen der Strömungsleitwand und den Längsseiten angeordnet sind, deren Rotationsachsen parallel zur Strömungsleitwand orientiert sind.

Die gasdichte Abdeckung des erfindungsgemäßen Erdbeckenfermenters kann aus mehreren Teilen, z.B. einer Membran und einem starren Teil, einer Bedienbrücke, einem Bedienplateau gebildet sein, wobei vorgesehen sein kann, dass die gasdichte Abdeckung durch zumindest eine Außenmembran und zumindest eine Innenmembran gebildet ist.

Zur Beheizung des Mediums kann innerhalb der Strömungsleitwand oder an deren Außenseite ein Heizelement angeordnet sein. Auf diese Weise kann die Beheizung des Mediums auch in einem Innenbereich des Erdbeckens erfolgen, wobei das Medium aufgrund der Eigenschaften der Strömungsleitwand an dieser vorbeigeführt wird und daher der Wärmeübergang auch auf der gesamten Höhe des Beckenquerschnitts sehr vorteilhaft ist.

Eine weitere Funktion der Strömungsleitwand kann darin bestehen, dass die Strömungsleitwand an zumindest einer Stelle ein Auflager zur Befestigung eines oder mehrerer Gurtnetze ausbildet, von welcher die zumindest eine Innenmembran, die Teil der gasdichten Abdeckung ist, im drucklosen Zustand vom Beckenboden beabstandet gehalten wird. Somit muss keine gesonderte Stütz- oder Haltevorrichtung für die Gurtnetze vorgesehen sein, die z.B. im Falle einer schneebedeckten Außenmembran sehr hohe Lasten aufnehmen müssen.

Das oder die zum Einsatz gelangenden Rührwerke können entweder im mittleren Bereich des Becken oder am Rand desselben angeordnet sein. Bei einer Montage am Rand ist es von Vorteil, wenn das Rührwerk einerseits gut zugänglich ist und andererseits auf einfache Weise höhenverstellbar angebracht ist. Der Antrieb des Rührwerks kann dabei außerhalb der gasdichten Abdeckung gelagert und die Rührschaufeln können durch eine durch die gasdichte Abdeckung hindurchverlaufende Welle angetrieben sein.

In weiterer Ausbildung der Erfindung kann dabei vorgesehen sein, dass das zumindest eine Rührwerk entlang einer schräg verlaufenden Stütze höhenverstellbar ist, wobei die Höhenverstellung mittels eines Einholseils, das mit einem Ende am Rührwerk angebracht ist und welches durch Einholen eine Bewegung des Rührwerks nach oben bewirkt, sowie eines Rückholseils, das mit einem Ende am Rührwerk angebracht ist und welches über zumindest eine im Bereich des unteren Endes der schräg verlaufenden Stütze angeordneten Umlenkrolle geführt ist, sodass durch Einholen desselben eine Bewegung des Rührwerks nach unten bewirkt wird.

Nachfolgend wird die Erfindung anhand der in den beigeschlossenen Zeichnungen dargestellten Ausführungsbeispiele eingehend erläutert. Es zeigt dabei
Fig. 1 eine Draufsicht auf ein Ausführungsbeispiel des erfindungsgemäßen Erdbeckenfermenters;
Fig.2 einen Längsschnitt entlang der Linie AA durch den Erdbeckenfermenter gemäß Fig.1;
Fig. 3 einen Querschnitt entlang der Linie BB durch den Erdbeckenfermenter gemäß Fig.1;
Fig.4 einen Querschnitt entlang der Linie CC durch den Erdbeckenfermenter gemäß Fig. 1;
Fig. 5 einen teilweisen Schnitt durch ein weiteres Ausführungsbeispiel eines erfindungsgemäßen Erdbeckenfermenters;

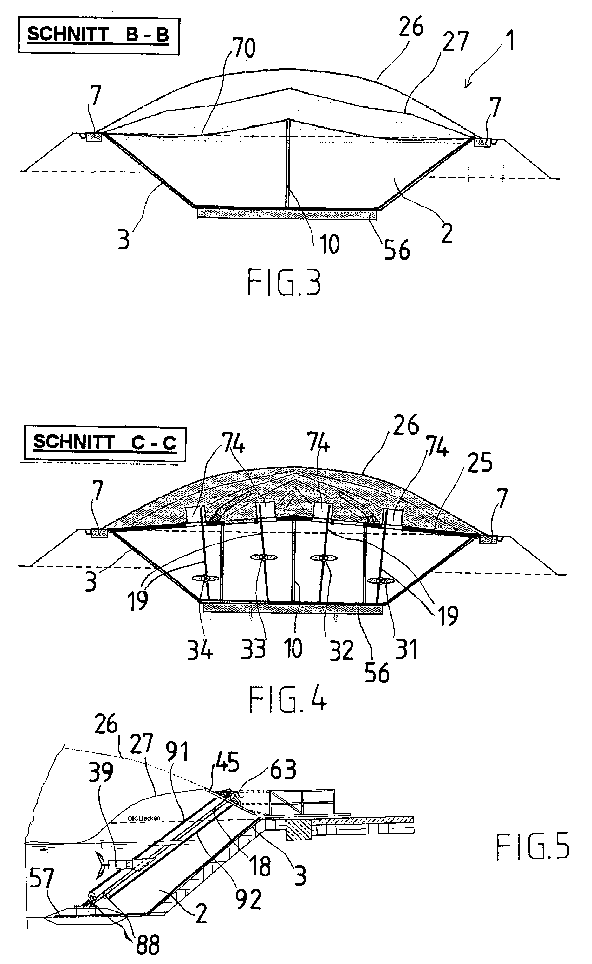

Fig.1, 2, 3 und 4 zeigen einen Erdbeckenfermenter 1 mit einem abgesenkten Beckenboden 2, der mit einer Dichtungsschicht 3 abgedeckt ist und ein Erdbecken ausbildet. Der Beckenboden weist dabei einen im Wesentlichen waagrechten Beckengrund auf, von dem sich Wandschrägen zu einer umlaufenden Beckenkrone 7 erstrecken (Fig.3 und 4).

An der Beckenkrone 7 ist eine gasdichte Abdeckung bereichsweise festgelegt, die sich im Wesentlichen aus zwei um die Nebenachse des Erdbeckens 2 spiegelsymmetrisch angeordneten Teilen zusammensetzt, die jeweils aus einer Außenmembran 26 und einer Innenmembran 27 bestehen. Die beiden Außenmembranen 26 und die beiden Innenmembranen 27 sind entlang ihres Umfangs an der Beckenkrone 7 sowie an beiden Seiten einer sich entlang der Becken-Nebenachse erstreckenden, im Inneren des Erdbeckens 2 durch eine Stützkonstruktion 85 getragene Bedienbrücke 25 gasdicht geklemmt. In bekannter Weise wird jeweils durch einen zwischen der Innenmembran 27 und der Außenmembran 26 eingeprägten, geregelten Stützdruck der Zustand der Außenmembran 26 weitgehend unabhängig vom augenblicklichen Ausdehnungszustand der Innenmembran 27, der wiederum von der jeweils gespeicherten Gasmenge im Inneren des Erdbeckenfermenters 1 abhängt, aufrecht erhalten.

Im Betrieb wird das Erdbecken 2 des Erdbeckenfermenters 1 mit einem Medium, z.B. Gülle, über nicht dargestellte Zuleitungen - maximal bis zur Beckenkronenoberkante - befüllt und das Medium der Fermentation überlassen. Um die Innenmembranen 27 vor einem vollständigen Absinken auf den Beckenboden oder in das Medium zu bewahren sind Gurtnetze 70 gespannt, auf welchen die Innenmembranen 27 aufliegen können.

Das sich durch Fermentation ausbildende Gas wird in dem von der Innenmembran 27 nach oben hin begrenzten Raum zwischengespeichert und kann nach Bedarf über nicht dargestellte Ableitungen entnommen werden. Über weitere nicht dargestellte Ableitungen, die sich entweder im Boden befinden können oder von oben in das Erdbecken 2 eingebracht sind, kann das Medium nach erfolgter Fermentation wieder abgesaugt werden.

Zum Zwecke der besseren Durchmischung des im Erdbecken 2 befindlichen Mediums sind Rührwerke 31, 32, 33, 34, 35 und 36 angeordnet, die entweder vollkommen eingetaucht sind oder zumindest mit ihren Schaufeln in das Medium ragen und welche über geeignete Antriebseinheiten angetrieben sind.

Die entlang der Becken-Nebenachse angeordneten Tauch-Rührwerke 31, 32, 33 und 34 sind höhenverstellbar gelagert, sodass die Medienbewegung in verschiedenen Höhenschichten geschehen kann, und ihre Rotationsachsen sind in Längsrichtung bzw. parallel zur Hauptachse des Erdbeckenfermenters 1 ausgerichtet, wodurch die Rührwerksschaufeln eine Strömung in Längsrichtung bewirken. Die Tauch-Rührwerke 31, 32, 33 und 34 sind dabei auf Stützen 19 angeordnet, die mit ihrem oberen Ende an der Bedienbrücke 25 festgelegt sind (Fig.4).

Über gasdichte Schachtkästen 74 kann durch Öffnungen in der Bedienbrücke 25 hindurch auf die Tauch-Rührwerke 31, 32, 33 und 34 zugegriffen werden und deren Höhenverstellung betätigt werden.

Wie aus Fig. 1 ersichtlich erstreckt sich die Beckenkrone 7 an beiden Längsseiten des Erdbeckens 2 parallel und ist an den beiden Schmalseiten durch Halbkreise abgeschlossen.

Erfindungsgemäß ist innerhalb des Erdbeckens 2 eine vertikale Strömungsleitwand 10 vorgesehen, welche entlang eines Abschnitts eine Unterteilung des Querschnitts des Erdbeckens 2 bewirkt, sodass bei Betätigung der Tauch-Rührwerke 31, 32, 33 und 34 sich eine Medienströmung zu beiden Seiten der Strömungsleitwand 10 ausbilden kann.

In den Halbkreisbereichen der Beckenkrone 7 sind weitere Rührwerke 35, 36 jeweils zwischen der Strömungsleitwand 10 und den Längsseiten angeordnet, deren Rotationsachsen ebenfalls parallel zur Strömungsleitwand 10 orientiert sind. Die weiteren Rührwerke 35, 26 unterstützen die Ausbildung einer Medienströmung zu beiden Seiten der Strömungsleitwand 10.

Die Höhe der Strömungsleitwand 10 ist größer als der maximale Füllstand im Erdbecken 2, sodass eine Durchmischung über die volle Höhe des Erdbeckenquerschnitts gewährleistet ist.

Die Strömungsleitwand 10 bildet ein Auflager zur Befestigung der Gurtnetze 70 aus, von welchen die Innenmembranen 27, die Teil der gasdichten Abdeckung sind, im drucklosen Zustand vom Beckenboden beabstandet gehalten werden, um deren Verschmutzung zu vermeiden.

Als zusätzliche Möglichkeit der Beheizung des Mediums kann innerhalb der Strömungsleitwand 10 oder an deren Außenseite ein Heizelement angeordnet sein, das z.B. durch Heizungsrohrschlangen gebildet sein kann.

Fig.5 zeigt eine weitere Ausführungsform des erfindungsgemäßen Erdbeckenfermenters, bei der das Rührwerk 39 entlang einer schräg verlaufenden Stütze 18 höhenverstellbar ist. Die Stütze 18 ist am unteren Ende an einem Fundamentelement 57 fixiert und an ihrem oberen Ende an einer schräg in das Erdbecken 2 ragenden Platte 45 eines Bedienplateaus angeordnet.

Die Höhenverstellung geschieht mittels eines Einholseils 91, das mit einem Ende am Rührwerk 39 angebracht ist und welches durch Einholen eine Bewegung des Rührwerks 39 nach oben bewirkt, sowie mittels eines Rückholseils 92, das mit einem Ende am Rührwerk 39 angebracht ist und welches über zumindest eine im Bereich des unteren Endes der schräg verlaufenden Stütze 18 angeordneten Umlenkrolle 88 geführt ist, sodass durch Einholen desselben eine Bewegung des Rührwerks 39 nach unten bewirkt wird.

## Patentansprüche

1. Erdbeckenfermenter mit einem abgesenkten Beckenboden, der mit einer Dichtungsschicht (3) abgedeckt ist und ein Erdbecken (2) ausbildet, und an dessen Beckenkrone (7) zumindest eine gasdichte Abdeckung (26, 27) festgelegt ist, wobei Zu- und Ableitungen und zumindest ein Rührwerk (31, 32, 33, 34, 35, 36, 39) vorgesehen sind, und wobei vom Beckenboden sich Wandschrägen zu der umlaufenden Beckenkrone erstrecken, **dadurch gekennzeichnet, dass** innerhalb des Erdbeckens (2) zumindest eine Strömungsleitwand (10) vorgesehen ist, welche entlang eines Abschnitts eine Unterteilung des Querschnitts des Erdbeckens (2) bewirkt, sodass bei Betätigung des zumindest einen Rührwerks (31, 32, 33, 34, 35) zu beiden Seiten der Strömungsleitwand (10) sich eine Medienströmung ausbildet.

2. Erdbeckenfermenter nach Anspruch 1, **dadurch gekennzeichnet, dass** die Strömungsleitwand (10) vertikal ist.

3. Erdbeckenfermenter nach Anspruch 1 oder 2, **dadurch gekennzeichnet, dass** die Höhe der Strömungsleitwand (10) größer als der maximale Füllstand im Erdbecken (2) ist.

4. Erdbeckenfermenter nach Anspruch 1, 2 oder 3, **dadurch gekennzeichnet, dass** die zumindest eine Strömungsleitwand (10) sich in Längsrichtung des Erdbeckens (2) erstreckt.

5. Erdbeckenfermenter nach einem der Ansprüche 1 bis 4, **dadurch gekennzeichnet, dass** die zumindest eine Strömungsleitwand durch eine Strömungsleitwand (10) ausgebildet ist, die sich entlang der Hauptachse des Erdbeckens (2) erstreckt und den Querschnitt des Erdbeckens (2) in zwei gleiche Teile unterteilt, wobei zwischen den Längsenden der Strömungsleitwand und dem in Längsrichtung jeweils gegenüberliegenden Beckenwandbereich ein Strömungsquerschnitt freigestellt ist.

6. Erdbeckenfermenter nach Anspruch 5, **dadurch gekennzeichnet, dass** das zumindest eine Rührwerk durch jeweils zwei Tauch-Rührwerke (31, 32, 33, 34) zu beiden Seiten der Strömungsleitwand (10) ausgebildet ist, welche mit ihren Rotationsachsen parallel zur Strömungsleitwand (10) bzw. zur Hauptachse ausgerichtet sind.

7. Erdbeckenfermenter nach Anspruch 6, **dadurch gekennzeichnet, dass** die Tauch-Rührwerke (31, 32, 33, 34) innerhalb des Erdbeckens (2) auf Stützen (19) höhenverstellbar angeordnet sind, die mit ihren oberen Enden auf einer Bedienbrücke (25) aufgelagert sind, welche Bedienbrücke (25) sich entlang der Nebenachse des Erdbeckens (2) über das Erdbecken (2) spannt und Teil der gasdichten Abdeckung bildet.

8. Erdbeckenfermenter nach Anspruch 5, 6 oder 7, **dadurch gekennzeichnet, dass** die Beckenkrone (7) sich an beiden Längsseiten des Erdbeckens (2) parallel erstreckt und an den beiden Schmalseiten durch Halbkreise abgeschlossen ist, und dass in den Halbkreisbereichen weitere Rührwerke (35, 36) jeweils zwischen der Strömungsleitwand (10) und den Längsseiten angeordnet sind, deren Rotationsachsen parallel zur Strömungsleitwand (10) orientiert sind.

9. Erdbeckenfermenter nach einem der vorhergehenden Ansprüche, **dadurch gekennzeichnet, dass** die gasdichte Abdeckung durch zumindest eine Außenmembran (26) und zumindest eine Innenmembran (27) gebildet ist.

10. Erdbeckenfermenter nach Anspruch 9, **dadurch gekennzeichnet, dass** die Strömungsleitwand (10) an zumindest einer Stelle ein Auflager zur Befestigung eines oder mehrerer Gurtnetze (70) ausbildet, von welcher die zumindest eine Innenmembran (27), die Teil der gasdichten Abdeckung ist, im drucklosen Zustand vom Beckenboden beabstandet gehalten wird.

11. Erdbeckenfermenter nach einem der vorhergehenden Ansprüche, **dadurch gekennzeichnet, dass** zur Beheizung des Mediums innerhalb der Strömungsleitwand (10) oder an deren Außenseite ein Heizelement angeordnet ist.

12. Erdbeckenfermenter nach einem der vorhergehenden Ansprüche, **dadurch gekennzeichnet, dass** das zumindest eine Rührwerk (39) entlang einer schräg verlaufenden Stütze (18) höhenverstellbar ist, wobei die Höhenverstellung mittels eines Einholseils (91), das mit einem Ende am Rührwerk (39) angebracht ist und welches durch Einholen eine Bewegung des Rührwerks (39) nach oben bewirkt, sowie eines Rückholseils (92), das mit einem Ende am Rührwerk (39) angebracht ist und welches über zumindest eine im Bereich des unteren Endes der schräg verlaufenden Stütze (18) angeordneten Umlenkrolle (88) geführt ist, sodass durch Einholen desselben eine Bewegung des Rührwerks (39) nach unten bewirkt wird.

## Claims

1. A ground-basin fermenter having a lowered basin bottom, which is covered by a sealing layer (3) and forms a ground basin (2), and which has at least one gas-tight cover (26, 27) attached to its basin rim (7), wherein supply and discharge lines and at least one stirring device (31, 32, 33, 34, 35, 36, 39) are provided, and wherein inclined walls extend from the basin bottom to the circumferential basin rim; **characterized in that** at least one flow-guide wall (10) is provided within the ground basin (2), resulting in a division of the cross section of the ground basin (2) along a portion thereof so that upon actuation of the at least one stirring device (31, 32, 33, 34, 35) a medium flow will form on both sides of the flow-guide wall (10).

2. The ground basin fermenter according to claim 1, **characterized in that** the flow-guide wall (10) is vertical.

3. The ground basin fermenter according to claim 1 or 2, **characterized in that** the height of the flow-guide wall (10) is larger than the maximum filling level of the ground basin (2).

4. The ground basin fermenter according to claim 1, 2 or 3, **characterized in that** the at least one flow-guide wall (10) extends in the longitudinal direction of the ground basin (2).

5. The ground basin fermenter according to any of the claims 1 to 4, **characterized in that** the at least one flow-guide wall is formed by a flow-guide wall (10) which extends along the main axis of the ground basin (2) and divides the cross section of the ground basin (2) into two equal parts, wherein a flow area is left clear between the longitudinal ends of the flow-guide wall and the respective basin wall regions longitudinally opposed thereto.

6. The ground basin fermenter according to claim 5, **characterized in that** the at least one stirring device is formed by at least two submersed stirring devices (31, 32, 33, 34) on each side of the flow-guide wall (10), which have their rotation axes arranged parallel to the flow-guide wall (10) and to the main axis, respectively.

7. The ground basin fermenter according to claim 6, **characterized in that** the submersed stirring devices (31, 32, 33, 34) within the ground basin (2) are arranged on pillars (19) in a height-adjustable manner, the upper ends of the pillars resting on an operating bridge (25), which operating bridge (25) extends across the ground basin (2) along the minor axis of the ground basin (2) and forms a part of the gas-tight cover.

8. The ground basin fermenter according to claim 5, 6 or 7, **characterized in that** the basin rim (7) extends parallely on both longitudinal sides of the ground basin (2) and is closed by semicircles on both narrow sides, and **in that** within the semicircular regions further stirring devices (35, 36), whose rotation axes are oriented in parallel to the flow-guide wall (10), are each arranged between the flow-guide wall (10) and the longitudinal sides.

9. The ground basin fermenter according to one of the previous claims, **characterized in that** gas-tight cover is formed by at least one outer membrane (26) and at least one inner membrane (27).

10. The ground basin fermenter according to claim 9, **characterized in that** the flow-guide wall (10) forms a bearing for attaching one or more cargo nets (70) at at least one point, by which the inner membrane (27), which is a part of the gas-tight cover, is held at a distance from the basin bottom in an unpressurized state.

11. The ground basin fermenter according to any of the previous claims, **characterized in that** a heating element is arranged within the flow-guide wall (10) or on its outer side to heat the medium.

12. The ground basin fermenter according to any of the previous claims, **characterized in that** the at least one stirring device (39) is height-adjustable along an inclined pillar (18), the height adjustment being provided by a pull-in rope (91), which is attached to the stirring device (39) with one end, and which, when pulled, causes an upward movement of the stirring device (39), and a return rope (92), which is attached to the stirring device (39) with one end and which is guided via at least one guide roller (88) arranged in the region of the lower end of the inclined pillar (18), so that pulling it causes a downward movement of the stirring device (39).

## Revendications

1. Fermenteur à bassin enterré rabaissé, recouvert par une couche d'étanchéité (3) et formant un bassin enterré (2), sur la crête (7) de son bassin étant fixé au moins une couverture (26, 27) étanche aux gaz, des conduites d'amenée et d'évacuation et au moins un agitateur (31, 32, 33, 34, 35, 36, 39) étant prévus et des parois inclinées s'étendant du fond du bassin vers la crête périphérique du bassin, **caractérisé en ce que** dans le bassin enterré (2) est prévu au moins une paroi (10) de guidage d'un courant, qui cause une répartition de la section du bassin enterré (2) le long d'une portion, de telle sorte qu'un courant d'un médium est formé des deux côtés de la paroi (10) de guidage du courant lors de l'activation d'au moins un agitateur (31, 32, 33, 34, 35).

2. Fermenteur à bassin enterré selon la revendication 1, **caractérisé en ce que** la paroi (10) de guidage du courant est verticale.

3. Fermenteur à bassin enterré selon la revendication 1 ou 2, **caractérisé en ce que** la hauteur de la paroi (10) de guidage du courant est supérieure au niveau maximal du bassin enterré (2).

4. Fermenteur à bassin enterré selon la revendication 1, 2 ou 3, **caractérisé en ce que** l'au moins une paroi (10) de guidage du courant s'étend dans le sens de la longueur du bassin enterré (2).

5. Fermenteur à bassin enterré selon une des revendications 1 à 4, **caractérisé en ce que** l'au moins une paroi de guidage du courant est formé par une paroi (10) de guidage du courant, qui s'étend le long de l'axe principal du bassin enterré (2) et repartit la section du bassin enterré (2) en deux parties égales, une section de courant étant laissée libre entre les extrémités longitudinales de la paroi de guidage du courant et la zone de la paroi du bassin respectivement opposée dans le sens de sa longueur.

6. Fermenteur à bassin enterré selon la revendication 5, **caractérisé en ce que** l'au moins un agitateur est formé par respectivement deux agitateurs à immersion (31, 32, 33, 34) des deux côtés de la paroi (10) de guidage du courant, lesquels sont orientés parallèlement à la paroi (10) de guidage du courant et/ou à l'axe principal avec leurs axes de rotation.

7. Fermenteur à bassin enterré selon la revendication 6, **caractérisé en ce que** les agitateurs à immersion (31, 32, 33, 34) sont disposés dans le bassin enterré (2) de manière réglable en hauteur sur des supports (19), qui reposent avec leurs extrémités supérieures sur un pont de commande (2), ledit pont de commande (2) s'étendant le long de l'axe secondaire du bassin enterré (2) à travers le bassin enterré (2) et formant une partie de la couverture étanche aux gaz.

8. Fermenteur à bassin enterré selon la revendication 5, 6 ou 7, **caractérisé en ce que** la crête du bassin (7) s'étend parallèlement des deux côtés du bassin enterré (2) et est terminée au niveau des deux côtés étroits par des demi-cercles, et **en ce que** dans les zones de demi-cercle sont positionnées d'autres agitateurs (35, 36), respectivement entre la paroi (10) de guidage du courant et les côtés longitudinales, dont les axes de rotation sont orientés parallèlement à la paroi (10) de guidage du courant.

9. Fermenteur à bassin enterré selon une des revendications précédentes, **caractérisé en ce que** la couverture étanche aux gaz est formée par au moins une membrane extérieure (26) et au moins une membrane intérieure (27).

10. Fermenteur à bassin enterré selon la revendication 9, **caractérisé en ce que** la paroi (10) de guidage de courant forme une base pour fixer un ou plusieurs filets à sangles (70) dans au moins un endroit, dans lequel l'au moins une membrane (27), faisant partie de la couverture étanche aux gaz, est maintenue à une distance du fond du bassin à l'état sans pression.

11. Fermenteur à bassin enterré selon une des revendications précédentes, **caractérisé en ce que** un élément de chauffage est positionné dans la paroi (10) de guidage du courant ou sur sa face extérieure pour chauffer le médium.

12. Fermenteur à bassin enterré selon une des revendications précédentes, **caractérisé en ce que** l'au moins un agitateur (39) peut être réglé en hauteur le long d'un support (18) incliné, le réglage en hauteur étant effectué par une corde de rattrapage (91), qui est fixée à l'agitateur (39) à une extrémité et qui cause un mouvement de l'agitateur (39) vers le haut quand elle est tirée, ainsi que d'une corde de retour (92), qui est fixée à l'agitateur (39) à une extrémité et qui est guidée par au moins une poulie de déviation (88), positionnée dans la zone de l'extrémité inférieure du support (18) incliné, de telle sorte qu'elle cause un mouvement de l'agitateur (39) vers le bas quand elle est tirée.
